(19) Europäisches Patentamt
European Patent Office
Office européen des brevets

(11) Veröffentlichungsnummer: **0 323 402 B1**

(12) # EUROPÄISCHE PATENTSCHRIFT

(45) Veröffentlichungstag der Patentschrift: **05.01.94**

(51) Int. Cl.[5]: **C07D 249/20**

(21) Anmeldenummer: **88810862.8**

(22) Anmeldetag: **14.12.88**

Die Akte enthält technische Angaben, die nach dem Eingang der Anmeldung eingereicht wurden und die nicht in dieser Patentschrift enthalten sind.

(54) **Verfahren zur Herstellung von flüssigen alkylierten 2-(2-Hydroxyphenyl)-benztriazol-Gemischen.**

(30) Priorität: **23.12.87 CH 5034/87**

(43) Veröffentlichungstag der Anmeldung:
**05.07.89 Patentblatt 89/27**

(45) Bekanntmachung des Hinweises auf die Patenterteilung:
**05.01.94 Patentblatt 94/01**

(84) Benannte Vertragsstaaten:
**BE CH DE ES FR GB IT LI**

(56) Entgegenhaltungen:
**US-A- 3 639 431**
**US-A- 4 587 346**

(73) Patentinhaber: **CIBA-GEIGY AG**
**Klybeckstrasse 141**
**CH-4002 Basel(CH)**

(72) Erfinder: **Stegmann, Werner, Dr.**
**Unter der Sonnhalde 9**
**CH-4410 Liestal(CH)**
Erfinder: **Luisoli, Reto**
**Stutzring 3**
**CH-4434 Hölstein(CH)**

**EP 0 323 402 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein verbessertes Verfahren zur Herstellung von flüssigen Isomerengemischen von 2-(2-Hydroxyphenyl)-benztriazolen durch Alkylierung von 2-(2-Hydroxy-5-methylphenyl)-benztriazol mit $C_8$-$C_{14}$-$\alpha$-Olefinen sowie die nach diesem Verfahren erhältlichen Gemische.

UV-Absorber der 2-(2-Hydroxyphenyl)-benztriazol-Reihe sind als sehr wirksame Lichtschutzmittel für organische Materialien verschiedenster Art seit langem bekannt und im Handel gut eingeführt. Beispiele für solche UV-Absorber, deren Herstellung und Anwendungsmöglichkeiten sind beispielsweise den US-A 3,004,896, 3,055,896, 3,072,585, 3,074,910, 3,189,615 und 3,230,194 zu entnehmen.

Für manche Anwendungen besitzen solche Benztriazole jedoch nur eine begrenzte Verträglichkeit und weisen eine Tendenz zum Ausschwitzen, Sublimieren und/oder Verflüchtigen auf, beispielsweise auch, wenn die Verarbeitung bei höheren Temperaturen erfolgen muss. Um dieses Verhalten zu verbessern, wurden Modifikationen im Substitutionsmuster der genannten Benztriazole vorgenommen. Siehe z.B. US-A 4,283,327, 4,278,590 und 4,383,863.

In neuerer Zeit wurde aus den oben erwähnten Gründen die Suche nach bei Raumtemperatur flüssigen 2-(2-Hydroxyphenyl)-benztriazolen intensiviert, da diese insbesondere auch Vorteile bei der Einarbeitung in bestimmte Substrate, z.B. in Photomaterialien, aufweisen. Siehe dazu die US-A 3,983,132, 4,096,242, 4,129,521 sowie die EP-A-57160.

In den US-A 4,587,346 und 4,675,352 sind Isomerenmischungen von höher alkyliertem 2-(2-Hydroxy-5-methylphenyl)-benztriazol und ein Verfahren zu deren Herstellung beschrieben. Diese Mischungen sind bei Raumtemperatur flüssig und weisen eine Reihe von hervorragenden applikatorischen Eigenschaften in diversen Substraten auf, wie sie in den beiden genannten Patenten beschrieben sind. Das darin beschriebene Herstellungsverfahren genügt in mancher Hinsicht nicht vollständig den Anforderungen an eine grosstechnische Herstellung der genannten Produkte. Es bestand daher Bedarf nach einem einfachen, kostengünstigen und in grossem Massstab realisierbaren Verfahren, das die gewünschten Produkte in hoher Ausbeute und guter Reinheit zu liefern imstande ist.

Es wurde nun ein Verfahren gefunden, das diesen Anforderungen entspricht, das das Produkt in überraschend hoher Reinheit und Ausbeute liefert. Die Lösung der gestellten Aufgabe wurde durch die Einhaltung von bestimmten Verfahrensparametern bei der Alkylierung in Verbindung mit einer spezifischen Aufarbeitungsmethode des Reaktionsgemisches erreicht.

Das erfindungsgemässe Verfahren zur Herstellung eines bei Raumtemperatur flüssigen 2-(2-Hydroxyphenyl)-benztriazol-Gemisches durch Alkylierung von 2-(2-Hydroxy-5-methylphenyl)-benztriazol mit einem $C_8$-$C_{14}$-$\alpha$-Olefin in der Schmelze bei erhöhter Temperatur in Gegenwart einer Sulfonsäure als Katalysator ist dadurch gekennzeichnet, dass man pro Mol 2-(2-Hydroxy-5-methylphenyl)-benztriazol mindestens 0,5 Mol des Säurekatalysators und 1,5-3 Mol $\alpha$-Olefin einsetzt, die Alkylierung bei 165-190° durchführt, die Rohproduktphase (obere Phase) von der Sulfonsäurephase trennt und erstere mit Hilfe eines Dünnschichtverdampfers, eines Dünnschichtverdampfers mit Destillationskolonne oder einer Kurzwegdestillationsapparatur destillative aufarbeitet.

Als saurer Katalysator wird eine Sulfonsäure verwendet. Es kann sich dabei um aromatische oder vorzugsweise aliphatische Sulfonsäuren handeln. Beispiele dafür sind Benzolsulfonsäure, Toluolsulfonsäuren, wie p-Toluolsulfonsäure, Methansulfonsäure und Ethansulfonsäure. Für praktische Belange ist Methansulfonsäure zu bevorzugen.

Die Reaktionstemperatur liegt vorzugsweise bei 170-180°C.

Die Menge an eingesetztem Sulfonsäure-Katalysator beträgt mindestens 0,5 Mol pro Mol 2-(2-Hydroxy-5-methylphenyl)-benztriazol. Die obere Grenze der Katalysatorkonzentration ist nicht kritisch, da auch bei hohen Sulfonsäuremengen gute Ergebnisse erzielt werden. Die Obergrenze wird sicher durch ökonomische Ueberlegungen bestimmt. So sind Katalysatormengen von 0,5-10 Mol/Mol Ausgangsprodukt zweckmässig. Vorzugsweise setzt man mindestens 0,8 Mol Sulfonsäure ein, z.B. 0,8-5 Mol. Besonders vorteilhaft und ökonomisch lässt sich das Verfahren mit etwa 1 Mol Sulfonsäure pro Mol Benztriazol-Ausgangsprodukt durchführen.

Die bevorzugte Menge an Alkylierungsmittel ($\alpha$-Olefin) liegt im Bereich von 2-3, z.B. 2,2-2,7, insbesondere bei etwa 2,5 Mol pro Mol 2-(2-Hydroxyphenyl)-benztriazol.

Als Alkylierungsmittel können sowohl geradkettige als auch verzweigte $\alpha$-Olefine verwendet werden. In der Praxis werden zweckmässigerweise im Handel erhältliche Isomerengemische von $\alpha$-Olefinen eingesetzt. Wie weiter unten noch ausgeführt wird, findet bei der Alkylierung ohnehin eine teilweise Isomerisierung der Alkylkette statt. Vorzugsweise wird die Alkylierung mit $C_{10}$-$C_{14}$-$\alpha$-Olefinen durchgeführt, insbesondere mit 1-Dodecen, wobei letzteres geradkettig oder verzweigt sein oder ein Isomerengemisch darstellen kann.

In einer besonders zweckmässigen Verfahrensvariante wird das Alkylierungsmittel kontinuierlich zur Reaktionsmischung zudosiert, z.B. mit Hilfe einer Dosierpumpe, und besonders bevorzugt erfolgt die Einleitung des Olefins unter die Oberfläche der Reaktionsmischung. Durch die dosierte Zugabe des Alkylierungsmittels wird insbesondere die Bildung von Nebenprodukten, wie Oligomeren der $\alpha$-Olefine, weiter reduziert.

Ferner ist es von Vorteil, für eine gute Durchmischung des Reaktionsgemisches zu sorgen. Für diesen Zweck empfiehlt sich der Einsatz einer leistungsfähigen Rührvorrichtung. Ebenfalls zweckmässig ist es, die Reaktion unter Inertgasatomsphäre, z.B. unter Stickstoff, durchzuführen.

Die Reaktion wird ohne Zugabe von eigentlichen Lösungsmitteln in der Schmelze durchgeführt. Als Lösungs- bzw. Verdünnungsmittel kann das $\alpha$-Olefin oder/und das durch die Alkylierung gebildete Gemisch der Endprodukte fungieren.

Nach beendigter Alkylierungsreaktion (die Reaktionszeit kann je nach gewählten Verfahrensparametern schwanken, liegt jedoch im allgemeinen zwischen 0,5 und 15, z.B. 3 und 10 Stunden) wird vom 2-phasigen Reaktionsgemisch die Rohproduktphase (obere Phase) abgetrennt und einem spezifischen destillativen Aufarbeitungsprozess unterworfen.

Vor der eigentlichen destillativen Aufarbeitung ist es zweckmässig, die Rohproduktphase zur Entfernung von Verunreinigungen zu waschen (extrahieren). Dies kann z.B. durch ein- oder mehrmaliges, z.B. 1 bis 5-maliges Waschen mit Wasser oder Natriumhydrogencarbonatlösung erfolgen. Insbesondere ist eine Extraktion mit letzterer und nachfolgende Extraktion mit Wasser vorteilhaft. Die vereinigten Waschextrakte werden vorzugsweise zur Sulfonsäurephase zum Zweck der Rückgewinnung von nicht umgesetztem Ausgangsprodukt zugegeben (siehe unten).

Nach erfolgter Extraktion empfiehlt es sich, die Rohproduktphase vor der eigentlichen destillativen Aufarbeitung zu entfärben. Dies kann durch Zusatz von üblichen Entfärbungsmitteln wie Aktivkohle und insbesondere Bleicherden geschehen. Ferner ist es vorteilhaft, restliches Wasser aus der Rohproduktphase durch Andestillieren zu entfernen. Nach Klärfiltration zur Entfernung des Entfärbungsmittels kann die eigentliche destillative Aufarbeitung des Rohproduktes erfolgen. Diese Aufarbeitung kann selbstverständlich auch ohne die fakultativen Schritte Extraktion, Entfärben und Entfernung des Wassers erfolgen, d.h. die aus dem Reaktionsgemisch abgetrennte Rohproduktphase kann direkt der destillativen Aufarbeitung unterworfen werden.

Letztere wird mit Hilfe eines Dünnschichtverdampfers, eines Dünnschichtverdampfers mit Destillationskolonne oder einer Kurzwegdestillationsapparatur durchgeführt. Darin werden zunächst Reste des eingesetzten $\alpha$-Olefins, bei der Reaktion als Nebenprodukte gebildete Olefin-Oligomere und schliesslich Reste von 2-(2-Hydroxy-5-methylphenyl)-benztriazol abdestilliert. Das zurückbleibende flüssige Produkt kann bereits als solches verwendet werden. Es ist jedoch vorteilhaft, es zur weiteren Reinigung in der gleichen oder in einer anderen Destillationsapparatur der beschriebenen Art zu destillieren. Man erhält so ein sehr reines Produkt.

Um das Verfahren noch wirtschaftlicher zu machen, ist es zweckmässig, nicht umgesetztes Benztriazol-Ausgangsprodukt aus der Reaktionsmischung zurückzugewinnen und wieder in der Alkylierungsreaktion einzusetzen. Zu diesem Zweck wird in der von der Rohproduktphase abgetrennten Sulfonsäurephase (untere Phase), die die Hauptmenge an nicht umgesetztem 2-(2-Hydroxy-5-methylphenyl)benztriazol enthält, letzteres durch kontrollierte Kristallisation ausgefällt, vorzugsweise durch Verdünnen mit Wasser oder/und durch Verdünnen mit den Waschextrakten aus der Extraktion der Rohproduktphase, sofern eine solche durchgeführt wurde. Die Ausfällung kann auch durch Neutralisation der Sulfonsäurephase bewerkstelligt werden bzw. vor der Verdünnung dieser Phase kann letztere auch neutralisiert werden. Das ausgeschiedene Benztriazol-Ausgangsprodukt kann in üblicher Weise abfiltriert, neutral gewaschen und gegebenenfalls getrocknet und im nächsten Alkylierungsansatz wiederverwendet werden, wobei eine Trocknung nicht unbedingt erforderlich ist.

Das bei der Destillation der Rohproduktphase anfallende erste Destillat enthält ebenfalls Benztriazol-Ausgangsprodukt, welches auch durch Kristallisation abgeschieden werden kann, z.B. durch Abkühlen, vorzugsweise auf etwa 0 °C. Nach Abfiltrieren, Waschen und gegegenenfalls Trocknen kann es ebenfalls wieder in die Alkylierungsreaktion zurückgeführt werden.

In der nachfolgenden schematischen Darstellung ist das erfindungsgemässe Verfahren anhand einer bevorzugten Ausführungsform (mit Rückgewinnung des Ausgangsproduktes) weiter verdeutlicht.

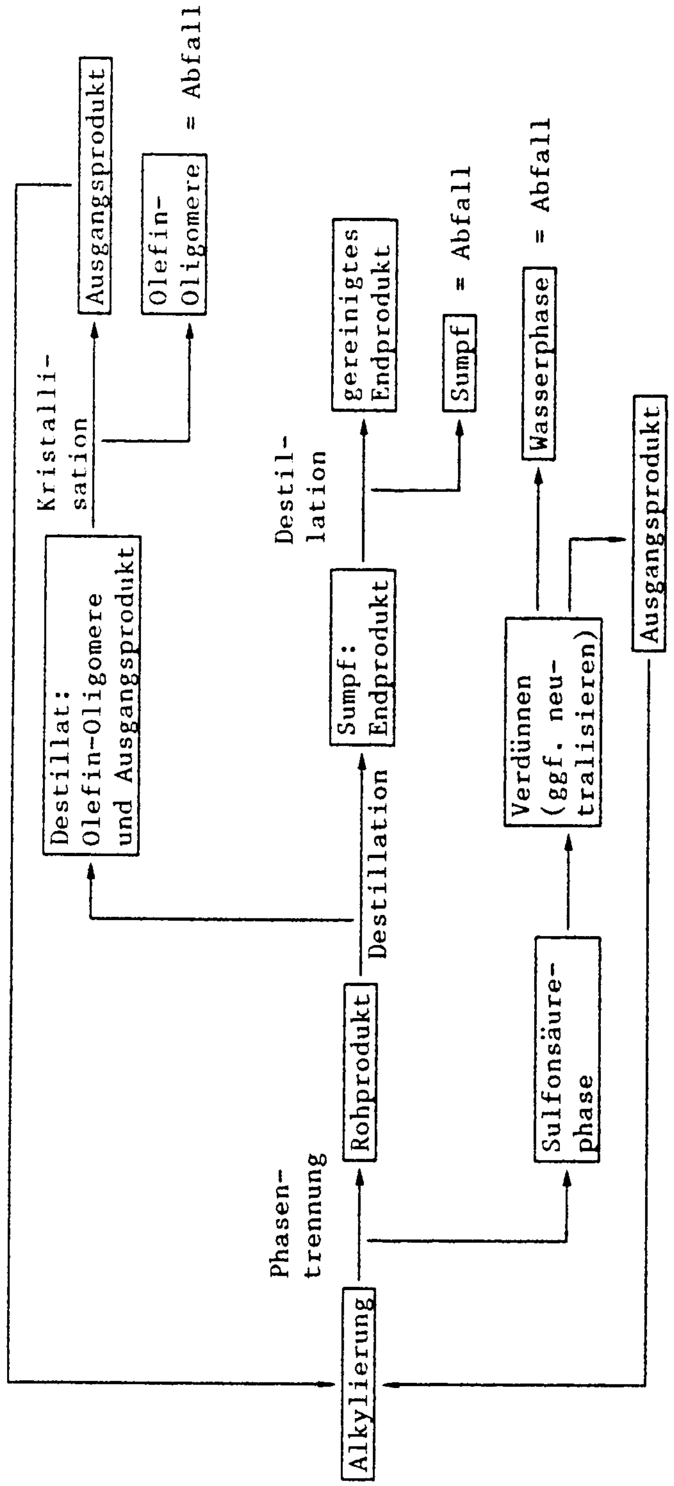


Ausgangsprodukt = 2-(2-Hydroxy-5-methylphenyl)-benztriazol

Endprodukt = Isomerengemisch von Verbindungen der Formel I

In einer weiteren bevorzugten Ausführungsform, insbesondere wenn Methansulfonsäure als Katalysator verwendet wird, wird nach der Alkylierung das Reaktionsgemisch mit Wasser verdünnt. Das Mengenverhältnis Wasser:Sulfonsäure im verdünnten Reaktionsgemisch kann in weiten Grenzen schwanken; es beträgt z.B. 0,5:1 bis 1:10, z.B. 0,8:1 bis 1:5, insbesondere etwa 1:1. Die Verdünnung mit Wasser hat zur Folge, dass praktisch die gesamte Restmenge an 2-(2-Hydroxy-5-methylphenyl)-benztriazol von der Sulfonsäurephase in die Rohproduktphase übergeht. Die Phasentrennung und die Aufarbeitung der Rohproduktphase erfolgt dabei wie vorstehend beschrieben. Die abgetrennte wässrige Sulfonsäurephase wird dann zweckmässig destillativ aufgearbeitet, z.B. in 3 Stufen, wobei in einem ersten Schritt der Hauptteil des Wassers, in einem zweiten Schritt verdünnte Methansulfonsäure (die zweckmässig wieder in die Destillation zurückgeführt wird), und in einem dritten Schritt reine Methansulfonsäure abdestilliert wird. Letztere kann problemlos wieder in einem neuen Alkylierungsansatz eingesetzt werden. Durch diese Verfahrensvariante entfällt einerseits der Rückgewinnungsschritt von nicht umgesetztem Benztriazol-Ausgangsprodukt aus der Sulfonsäurephase und andererseits kann die Sulfonsäure fast vollständig recycliert werden. Diese Verfahrensvariante kann etwa durch folgendes Schema dargestellt werden:

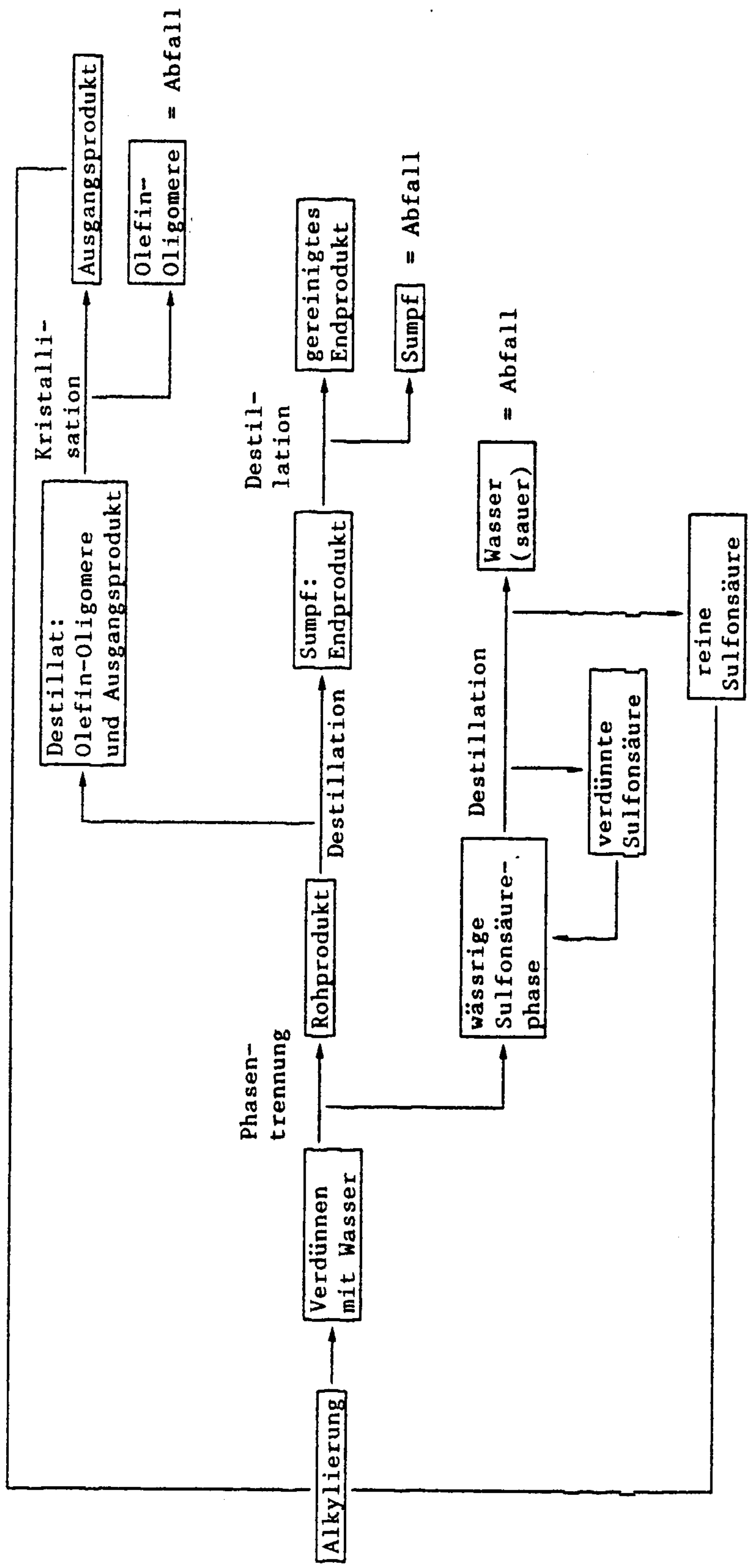

Das als Ausgangsprodukt eingesetzte 2-(2-Hydroxy-5-methylphenyl)-benztriazol ist bekannt und im Handel als UV-Absorber erhältlich.

Die erfindungsgemäss erhältlichen flüssigen alkylierten 2-(2-Hydroxy-phenyl)-benztriazol-Gemische sind wertvolle Lichtschutzmittel, die in einer Reihe von Substraten eingesetzt werden können, um letztere vor Schädigungen durch Lichteinwirkung zu schützen. Beispiele für solche Anwendungsmöglichkeiten sind den US-A- 4,587,346 und 4,675,352 zu entnehmen. Besonders vorteilhaft ist der Einsatz in Lacksystemen und insbesondere in photographischen Materialien.

Wie bereits erwähnt, handelt es sich bei den erfindungsgemäss erhältlichen Produkten um Gemische von Verbindungen. Die Produkte enthalten hauptsächlich Alkylierungsprodukte der Formel

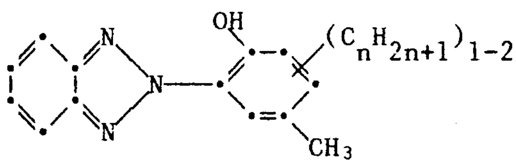

(I) ,

worin n eine Zahl von 8 bis 14 ist und der Alkylrest ein Gemisch aus mindestens 2, insbesondere mindestens 3, isomeren Resten darstellt. Durch die Alkylierung werden 1 bis 2 Alkylreste eingeführt. Die Hauptkomponenten enthalten nur einen langkettigen Alkylrest, der seinerseits natürlich ein Isomerengemisch aus mindestens 2 Isomeren ist. Bevorzugt ist n 10 bis 14, insbesondere 12.

Der(die) eingeführte(n) lange(n) Alkylrest(e) ist(sind) als solche(r) (ein) statische(s) Gemisch(e) aus zwei oder mehr isomeren Alkylketten. Abgesehen davon, dass man als α-Olefin Isomerenmischungen einsetzen kann, tritt bei der Alkylierung in jedem Fall eine Isomerisierung der Alkylkette ein. Insbesondere kann die Bindung des α-Olefins über die 1-Stellung oder die 2-Stellung erfolgen. Im ersten Fall resultiert ein α-unverzweigter, im zweiten Fall ein α-verzweigter Alkylrest.

Ein langer Alkylrest in Formel I steht vorzugsweise in 3-Stellung des Phenylrings (in ortho-Stellung zur OH-Gruppe). Das erfindungsgemäss erhältliche Gemisch kann aber auch noch geringe Mengen von anderen Reaktionsprodukten enthalten, die nicht durch die Formel I umfasst sind. So kann Alkylierung in Spuren auch im Benzorest des Benztriazolringes stattfinden, insbesondere Alkylierung mit kurzkettigen Spaltungsprodukten des eingesetzten Olefins. Besagter Benzorest kann damit z.B. eine oder mehrere Alkylgruppen mit weniger als 8 C-Atomen enthalten. Auch der Phenolrest kann in sehr geringem Ausmass durch solche kurzkettigen Alkylgruppen substituiert werden. Auf der anderen Seite kann im Reaktionsgemisch Oligomerisierung des Olefins eintreten. Solche Oligomere, insbesondere Dimere, können ebenfalls zur Alkylierung des Phenolringes führen, so dass auch Verbindungen entstehen, die an diesem Ring mit Alkylketten mit mehr als 14 C-Atomen, z.B. solchen mit 16 bis 28 C-Atomen substituiert sind.
Verbindungen dieses Typs können in wechselnden Anteilen im Endprodukt enthalten sein, beispielsweise bis zu 5 %, in Einzelfällen bis zu 10 %.

Wie bereits angedeutet, wird unter den Reaktionsbedingungen das eingesetzte Olefin oligomerisiert, insbesondere dimerisiert. Diese Oligomeren werden zwar bei der destillativen Reinigung zum grössten Teil abgetrennt, das Produkt kann aber noch solche Dimere bzw. Oligomere enthalten, beispielsweise in einer Menge von 0,1 bis 5, in Extremfällen bis 10 %.

Die quantitative Analyse des Reaktionsproduktes ist äusserst schwierig, so dass es nicht möglich ist, seine genaue mengenmässige Zusammensetzung zu bestimmen.

Wie bereits ausgeführt, ist aus den US-A 4,587,346 und 4,675,352 ein Basisverfahren zur Herstellung von Isomerenmischungen vom Typ der Formel I bekannt. Das dort konkret beschriebene Verfahren (siehe Beispiele 1-7 in diesen beiden Patentschriften) führt nur zu relativ niedrigen Ausbeuten und ist wegen der im grosstechnischen Massstab schwer durchführbaren Aufarbeitung durch Lösungsmittelextraktion in der Praxis nicht sehr günstig. Durch die erfindungsgemässe Kombination von spezifischen Verfahrensparametern und besonderer Aufarbeitungsmethode können diese Nachteile überwunden werden. Dabei ist es insbesondere als überraschend anzusehen, dass bei der destillativen Aufarbeitung der Produkteschmelze die Trennung der Olefin-Oligomeren von den Produkten der Formel I so glatt möglich ist, obwohl eine destillative Trennung mit anderen konventionellen Destillationsvorrichtungen nicht zum Ziel führt.

Mit Hilfe des erfindungsgemässen Verfahrens wird nicht nur eine hohe Ausbeute erreicht, sondern überraschenderweise auch Produkte, die sich für verschiedene Applikationen noch besser eignen als nach dem bekannten Verfahren hergestellte Produkte.

Bei Anwendung von bevorzugten Ausführungsformen, z.B. dosierungskontrollierte Zugabe des α-Olefins, wird die Bildung von Olefin-Oligomeren weiter unterdrückt und der Ueberschuss an α-Olefin kann

gesenkt werden. Der Umsatz ist besser und der Restgehalt an Ausgangsprodukt geringer. Wenn man die bevorzugte Rückgewinnung an nicht umgesetztem Ausgangsprodukt in Betracht zieht, erreicht man Ausbeuten bis nahe 100 % der Theorie.

Die nachfolgenden Beispiele erläutern das erfindungsgemässe Verfahren weiter. Teile- und Prozentangaben beziehen sich darin sowie in der übrigen Beschreibung auf das Gewicht, sofern nichts anderes angegeben ist.

Beispiel 1: Bei Raumtemperatur werden in einem 1,5 l Doppelmantelkolben mit Propellerrührer, Unterniveau-Dosiereinrichtung mit Dosierpumpe, Stickstoffeinleitung und mit einer Wasserstrahlpumpe verbundenem Destillationsaufsatz 225,2 g (1 Mol) 2-(2-Hydroxy-5-methylphenyl)-benztriazol und 96,1 g (1 Mol) Methansulfonsäure vorgelegt und mit Stickstoff inertisiert. Unter langsamem Rühren wird das Gemisch auf 175 °C erhitzt, wobei bei ca. 125 °C die Suspension in eine Lösung übergeht. Bei 175 °C werden unter kräftigem Rühren innerhalb 6 Stunden 420,8 g (2,5 Mol) n-Dodecen gleichmässig unter die Oberfläche des Gemisches zudosiert. Nach weiteren 30 Minuten Rühren bei 175 °C ist die Alkylierung beendet. Der Restgehalt an nicht umgesetztem 2-(2-Hydroxy-5- methylphenyl)-benztriazol wird dünnschichtchromatographisch zu 3-5%, entsprechend 15-20 % der ursprünglich eingesetzten Menge, bestimmt. Nach Abkühlen auf ca. 95 °C wird die untere, Methansulfonsäure und nicht umgesetztes Benztriazol-Ausgangsprodukt enthaltende, Phase abgetrennt. Die obere Phase (Rohproduktphase) wird zuerst mit 100 ml 4 %-iger Natriumhydrogencarbonatlösung und anschliessend zweimal mit 100 ml Wasser extrahiert. Die Extrakte werden vorsichtig zur abgetrennten Methansulfonsäurephase gegeben, wobei das gelöste 2-(2-Hydroxy-5-methylphenyl)-benztriazol auskristallisiert. Letzteres wird filtriert, neutral gewaschen und getrocknet. Man erhält so 25 g des Ausgangsproduktes, entsprechend 11,2 %, bezogen auf die eingesetzte Menge.

Die extrahierte Rohproduktphase wird nach Zugabe von 10 g Bleicherde (®Prolith Rapid) durch Andestillieren bis 120 °C und 5 mbar vollständig von Wasser befreit und durch Klärfiltration bei 100 °C entfärbt. Man erhält so ca. 570 g hellgelbes, klares Rohprodukt.

Dieses Rohprodukt wird nun auf einem 0,02 $m^2$ Dünnschichtverdampfer bei einer Manteltemperatur von 240 °C und einem Druck von 1-3 mbar so destilliert, dass der gaschromatographisch gemessene Gehalt an Dodecen-Oligomeren kleiner als 2 % und an 2-(2-Hydroxy-5-methylphenyl)-bentriazol kleiner als 0,2 % ist. Der Rückstand (Sumpf, ca. 320 g) wird schliesslich auf dem Dünnschichtverdampfer bei einer Manteltemperatur von 285 °C und einem Druck von 1-3 mbar destilliert (Kopftemperatur ca. 240 °C). Man erhält 315 g (Ausbeute: 80 % der Theorie) der gewünschten Mischung aus Verbidungen der Formel I mit n = 12 in Form eines hellgelben, flüssigen Produktes mit einem $n_D^{20}$ von 1,5677.

Das Destillat aus der ersten Destillation am Dünnschichtverdampfer wird in einem 350 ml Sulfierkolben mit Ankerrührer auf 0 °C abgekühlt, wodurch weitere 16 g (entsprechend 7 % der Theorie, bezogen auf die eingesetzte Menge) 2-(2-Hydroxy-5-methylphenyl)-benztriazol auskristallisieren, welche abfiltriert, gewaschen und getrocknet werden. Die Gesamtmenge an wiedergewonnenem Ausgangsprodukt beträgt somit ca. 18% der Theorie, bezogen auf die urpsrünglich eingesetzte Menge. Dieses Ausgangsprodukt kann im nächsten Reaktionsansatz wieder eingesetzt werden, wobei eine vorgängige Trocknung nicht erforderlich ist.

Beispiel 2: Bei Raumtemperatur werden in einem 1,5 l Doppelmantelkolben mit Propellerrührer, Unterniveau-Dosiereinrichtung mit Dosierpumpe, Stickstoffeinleitung und mit einer Wasserstrahlpumpe verbundenem Destillationsaufsatz 225,2 g (l Mol) 2-(2-Hydroxy-5-methylphenyl)-benztriazol und 96,1 g (1 Mol) Methansulfonsäure vorgelegt und mit Stickstoff inertisiert. Unter langsamem Rühren wird das Gemisch auf 175 °C erhitzt, wobei bei ca. 125 °C die Suspension in eine Lösung übergeht. Bei 175 °C werden unter kräftigem Rühren innerhalb 8 Stunden 420,8 g (2,5 Mol) n-Dodecen gleichmässig unter die Oberfläche des Gemische zudosiert. Nach weitern 30 Minuten Rühren bei 175 °C ist die Alkylierung beendet. Der Restgehalt an nicht umgesetztem 2-(2-Hydroxy-5-methylphenyl)-benztriazol wird dünnschichtchromatographisch zu max. 3 %, entsprechend 12-16 % der ursprünglich eingesetzten Menge, bestimmt. Nach Abkühlen auf ca. 90 °C werden innerhalb 10 Minuten 96 ml Wasser zugetropft. Nach 10-minütigem Rühren bei 90 °C wird die untere Methansulfonsäure enthaltende Phase vorsichtig abgetrennt (Aufarbeitung der letzteren siehe unten). Die obere Phase (Rohproduktphase) wird zuerst mit 100 ml 4%-iger Natriumhydrogencarbonatlösung und anschliessend einmal mit 100 ml Wasser extrahiert.

Die extrahierte Rohproduktphase wird nach Zugabe von 10 g Bleicherde (®Prolith Rapid) durch Andestillieren bis 120 °C und 5 mbar vollständig von Wasser befreit und durch Klärfiltration bei 120 °C entfärbt. Man erhält so ca. 610 g hellgelbes, klares Rohprodukt.

Dieses Rohprodukt wird nun auf einem 0,02 $m^2$ Dünnschichtverdampfer bei einer Manteltemperatur von 240 °C und einem Druck von 1-3 mbar so destilliert, dass der geschromatographisch gemessene Gehalt an Dodecen-Oligomeren kleiner als 2 % und an 2-(2-Hydroxy-5-methylphenyl)-benztriazol kleiner als 0,2 % ist. Der Rückstand (Sumpf, ca. 347 g) wird schiesslich auf dem Dünnschichtverdampfer bei einer Manteltemperatur von 285 °C und einem Druck von 1-3 mbar destilliert (Kopftemperatur ca. 240 °C). Man erhält 326 g (Ausbeute: 85 % der Theorie) der gewünschten Mischung aus Verbindungen der Formel I mit n- 12 in Form eines hellgelben, flüssigen Produktes mit einem $n_D^{20}$ von 1,5677.

Das Destillat aus der ersten Destillation am Dünnschichtverdampfer wird in einem 350 ml Sulfierkolben mit Ankerrührer auf 0 °C abgekühlt, wodurch ca. 27 g (entsprechend 12 % der Theorie, bezogen auf die eingesetzte Menge) 2-(2-Hydroxy-5-methylphenyl)-benztriazol auskristallisieren, welche abfiltriert und gewaschen werden. Die Gesamtmenge an wiedergewonnenem Ausgangsprodukt beträgt somit ca. 12 % der Theorie, bezogen auf die ursprünglich eingesetzte Menge. Dieses Ausgangsprodukt kann im nächsten Reaktionsansatz wieder eingesetzt werden, wobei eine vorgängige Trocknung nicht erforderlich ist.

In einem 250 ml Herzkolben mit Kapillare, 15 cm Vigreux-Kolonne, Destillationsaufsatz mit Kühler, Thielevorstoss und 100 ml Rundkolben, verbunden mit einer Vakuum-Drehschieberpumpe, werden ca. 185 g der abgetrennten wässrigen Methansulfonsäurephase vorgelegt. Nach Anlegen eines Vakuums von 100 mbar wird so lange erhitzt, bis bei einer Badtemperatur von ca. 155 °C und einer Kopftemperatur von ca. 55 °C praktisch kein Wasser mehr destilliert und die Kopftemperatur absinkt. Es werden so ca. 85 ml leicht saures Wasser abdestilliert (pH>3). Nach Wechseln der Vorlage wird das Vakuum bis auf ca. 2 mbar verbessert, wodurch ca. 7,5 g wässrige Methansulfonsäure (10-20 %) abdestillieren, welche der nächsten Destillation wieder zugesetzt werden können. Nach erneutem Wechseln der Vorlage werden bei einem Vakuum von ca. 2 mbar und einer Kopftemperatur von ca. 125-130 °C und einer Badtemperatur von 155-175 °C ca. 87 g reine Methansulfonsäure abdestilliert. Diese Methansulfonsäure entspricht in bezug auf Aussehen (leicht bräunlich) und Gehalt (99 %) neuer technischer Ware. Der Wassergehalt (0.5 % $H_2O$) liegt sogar tiefer als bei handelsüblicher Ware. Die erzielte Ausbeute beträgt ca. 90 % der bei der Alkylierung eingesetzten Menge Methansulfonsäure.

Diese Destillation kann in gleicher Weise auch in einem Sulfierkolben (batchweise) oder kontinuierlich in einer 2-stufigen Destillationsapparatur durchgeführt werden.

**Patentansprüche**

1. Verfahren zur Herstellung eines bei Raumtemperatur flüssigen 2-(2-Hydroxyphenyl)-benztriazol-Gemisches durch Alkylierung von 2-(2-Hydroxy-5-methylphenyl)-benztriazol mit einem $C_8$-$C_{14}$-$\alpha$-Olefin in der Schmelze bei erhöhter Temperatur in Gegenwart einer Sulfonsäure als Katalysator, dadurch gekennzeichnet, dass man pro Mol 2-(2-Hydroxy-5-methylphenyl)-benztriazol mindestens 0,5 Mol des Säurekatalysators und 1,5-3 Mol $\alpha$-Olefin einsetzt, die Alkylierung bei 165-190 °C durchführt, die Rohproduktphase (obere Phase) von der Sulfonsäurephase trennt und erstere mit Hilfe eines Dünnschichtverdampfers, eines Dünnschichtverdampfers mit Destillationskolonne oder einer Kurzwegdestillationsapparatur destillativ aufarbeitet.

2. Verfahren nach Anspruch 1, worin als Katalysator Methansulfonsäure eingesetzt wird.

3. Verfahren nach Anspruch 1, worin die Alkylierung bei 170-180 °C durchgeführt wird.

4. Verfahren nach Anspruch 1 oder 2, worin mindestens 0,8 Mol Sulfonsäure pro Mol 2-(2-Hydroxy-5-methylphenyl)-benztriazol eingesetzt werden.

5. Verfahren nach Anspruch 1, worin 2,2-2,7 Mol $\alpha$-Olefin pro Mol 2-(2-Hydroxy-5-methylphenyl)-benztriazol verwendet werden.

6. Verfahren nach einem der Ansprüche 1-5, worin die Alkylierung mit einem 1-Dodecen durchgeführt wird.

7. Verfahren nach Anspruch 1, worin das $\alpha$-Olefin zur Reaktionsmischung mit Hilfe einer Dosierungseinrichtung kontinuierlich zudosiert wird, vorzugsweise unter die Oberfläche der Reaktionsmischung.

8. Verfahren nach Anspruch 1, worin das Reaktionsgemisch vor der Phasentrennung mit Wasser verdünnt wird.

9. Verfahren nach einem der Ansprüche 1-8, worin die abgetrennte Rohproduktphase vor der destillativen Aufarbeitung gewaschen (extrahiert), entfärbt und durch Andestillieren von Wasser weitgehend befreit wird.

**10.** Verfahren nach Anspruch 1 oder 9, worin die eigentliche destillative Aufarbeitung darin besteht, zunächst nicht umgesetztes Ausgangsprodukt und $\alpha$-Olefin-Oligomere abzudestillieren und gegebenenfalls anschliessend das Produkt selbst in einer der genannten Apparaturen durch Destillation zu reinigen.

**11.** Verfahren nach einem der Ansprüche 1-7, worin für den Fall, dass das Reaktionsgemisch vor der Phasentrennung nicht verdünnt wurde, aus der abgetrennten Sulfonsäurephase nicht umgesetztes Benztriazol-Ausgangsprodukt zurückgewonnen und in die Alkylierungsstufe zurückgeführt wird.

**12.** Verfahren nach Anspruch 11, worin in der Sulfonsäurephase das 2-(2-Hydroxy-5-methylphenyl)-benztriazol durch kontrollierte Kristallisation abgeschieden wird, vorzugsweise durch Verdünnen mit Wasser oder/und Zugabe der Waschextrakte aus der Extraktion der Rohproduktphase.

**13.** Verfahren nach Anspruch 1, 9 oder 10, worin aus dem aus der Rohproduktphase abdestillierten Gemisch 2-(2-Hydroxy-5-methylphenyl)-benztriazol durch Kristallisation abgeschieden wird.

**14.** Verfahren nach Anspruch 8, worin nach der Abtrennung der Rohproduktphase aus der wässrigen Sulfonsäurephase durch destillative Aufarbeitung die Sulfonsäure wiedergewonnen wird.

**15.** Verfahren nach einem der Ansprüche 1 bis 14, worin die Hauptmenge des erhaltenen flüssigen 2-(2-Hydroxyphenyl)-benztriazol-Gemisches aus Verbindungen der Formel

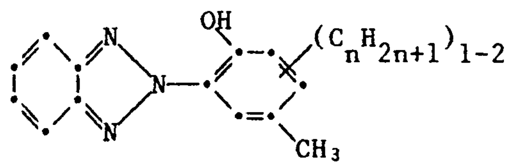

(I)

besteht, worin n eine Zahl von 8 bis 14 ist und der Alkylrest ein Gemisch aus mindestens zwei isomeren Resten darstellt.

**Claims**

**1.** A process for the preparation of a mixture of 2-(2-hydroxyphenyl)benzotriazoles which is liquid at room temperature by alkylating 2-(2-hydroxy-5-methylphenyl)benzotriazole with a $C_8$-$C_{14}\alpha$-olefin in the melt at elevated temperature, in the presence of a sulfonic acid as catalyst, which process comprises using not less than 0.5 mol of the acid catalyst and 1.5 - 3 mol of $\alpha$-olefin per mole of 2-(2-hydroxy-5-methylphenyl)benzotriazole, carrying out the alkylation at from 165-190 °C, separating the crude product phase (upper phase) from the sulfonic acid phase, and working up said crude product phase by distillation by means of a thin-film evaporator, a thin-film evaporator fitted with a distillation column, or a short-path distillation apparatus.

**2.** A process according to claim 1, wherein the catalyst used is methanesulfonic acid.

**3.** A process according to claim 1, wherein the alkylation is carried out at from 170 - 180 °C.

**4.** A process according to either claim 1 or claim 2, wherein not less than 0.8 mol of sulfonic acid is used per mole of 2-(2-hydroxy-5-methylphenyl)benzotriazole.

**5.** A process according to claim 1, wherein 2.2 - 2.7 mol of $\alpha$-olefin are used per mole of 2-(2-hydroxy-5-methylphenyl)benzotriazole.

**6.** A process according to any one of claims 1 to 5, wherein the alkylation is carried out with a l-dodecene.

**7.** A process according to claim 1, wherein the $\alpha$-olefin is metered continuously to the reaction mixture by means of a metering device, preferably beneath the surface of the reaction mixture.

8. A process according to claim 1, wherein the reaction mixture is diluted with water before phase separation.

9. A process according to any one of claims 1 to 8, wherein the separated crude product phase is washed (extracted) before the distillative work-up, decolorized, and substantially freed from water by initial distillation.

10. A process according to either claim 1 or claim 9, wherein the actual distillative work-up comprises first separating unreacted starting material and oligomers of the α-olefin by distillation and, if desired, subsequently purifying the product itself by distillation in an apparatus as mentioned.

11. A process according to any one of claims 1 to 7, wherein unreacted starting benzotriazole is recovered from the separated sulfonic acid phase and recycled to the alkylation step, if the reaction mixture has not been diluted before phase separation.

12. A process according to claim 11, wherein 2-(2-hydroxy-5-methylphenyl)benzotriazole is precipitated in the sulfonic acid phase by controlled crystallization, preferably by dilution with water and/or addition of the washing extracts from the extraction of the crude product phase.

13. A process according to any one of claims 1, 9 or 10, wherein 2-(2-hydroxy-5-methylphenyl)-benzotriazole is precipitated by crystallization from the mixture distilled from the crude product phase.

14. A process according to claim 8, wherein the sulfonic acid is recovered after separation of the crude product phase from the aqueous sulfonic acid phase by distillative work-up.

15. A process according to any one of claims 1 to 14, wherein the bulk of the liquid mixture of 2-(2-hydroxyphenyl)benzotriazoles obtained consists of compounds of the formula

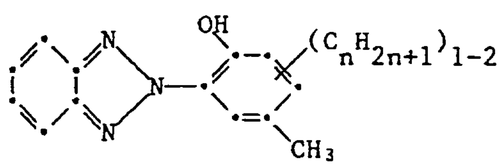

(I)

wherein n is a number from 6 to 14 and the alkyl radical is a mixture of at least two isomeric radicals.

**Revendications**

1. Procédé de préparation d'un mélange de 2-(2-hydroxyphényl)-benzotriazoles liquide à la température ambiante par alkylation du 2-(2-hydroxy-5-méthylphényl)-benzotriazole avec une α-oléfine en $C_8$-$C_{14}$, à l'état fondu, à température élevée, en présence d'un acide sulfonique comme catalyseur, caractérisé en ce qu'on utilise par mole de 2-(2-hydroxy-5-méthylphényl)-benzotriazole au moins 0,5 mol du catalyseur acide et 1,5-3 moles d'α-oléfine, en ce qu'on effectue l'alkylation à 165-190 °C, en ce qu'on sépare la phase produit brut (phase supérieure) de la phase acide sulfonique et en ce qu'on traite la première par distillation à l'aide d'un évaporateur à couche mince, d'un évaporateur à couche mince avec colonne de distillation ou d'un appareil de distillation moléculaire.

2. Procédé selon la revendication 1, dans lequel on utilise comme catalyseur l'acide méthane-sulfonique.

3. Procédé selon la revendication 1, dans lequel l'alkylation est effectuée à 170-180 °C.

4. Procédé selon les revendications 1 ou 2, dans lequel on utilise au moins 0,8 mole d'acide sulfonique par mole de 2-(2-hydroxy-5-méthylphényl)-benzotriazole.

5. Procédé selon la revendications 1, dans lequel on utilise 2,2-2,7 mol d'α-oléfine par mole de 2-(2-hydroxy-5-méthylphényl)-benzotriazole.

6. Procédé selon l'une des revendications 1 à 5, dans lequel on effectue l'alkylation avec un 1-dodécène.

7. Procédé selon la revendications 1, dans lequel on dose en continu l'α-oléfine dans le mélange réactionnel à l'aide d'un dispositif de dosage, de préférence sous la surface du mélange réactionnel.

8. Procédé selon la revendications 1, dans lequel le mélange réactionnel est dilué avec de l'eau avant la séparation des phases.

9. Procédé selon l'une des revendications 1 à 8, dans lequel la phase produit brut séparé est lavée (extraite) avant le traitement par distillation, décolorée et débarrassée dans une large mesure de l'eau par distillation.

10. Procédé selon les revendications 1 ou 9, dans lequel le traitement de distillation proprement dit consiste à distiller d'abord le produit de départ n'ayant pas réagi et les oligomères de l'α-oléfine, puis le cas échéant à purifier le produit lui-même par distillation dans l'un des appareils indiqués.

11. Procédé selon l'une des revendications 1 à 7, dans lequel, au cas où le mélange réactionnel n'a pas été dilué avant la séparation des phases, on récupère à partir de la phase acide sulfonique séparée le benzotriazole de départ n'ayant pas réagi et on le renvoie à l'étage d'alkylation.

12. Procédé selon la revendication 11, dans lequel, dans la phase acide sulfonique, le 2-(2-hydroxy-5-méthylphényl)-benzotriazole est séparé par cristallisation contrôlée, de préférence par dilution avec de l'eau et/ou addition des extraits aqueux provenant de l'extraction de la phase produit brut.

13. Procédé selon les revendications 1, 9 ou 10, dans lequel on sépare par cristallisation le mélange de 2-(2-hydroxy-5-méthylphényl)-benzotriazoles distillé à partir de la phase produit brut.

14. Procédé selon la revendication 8, dans lequel, après séparation de la phase produit brut de la phase acide sulfonique aqueuse, on récupère l'acide sulfonique par un traitement de distillation.

15. Procédé selon l'une des revendications 1 à 14, dans lequel la plus grande partie du mélange de 2-(2-hydroxyphényl)-benzotriazoles liquides obtenu est constituée de composés répondant à la formule :

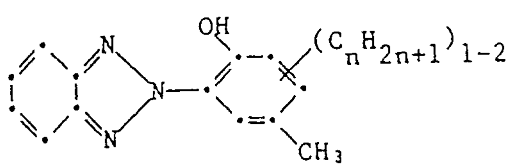

(I)

dans laquelle n est un nombre de 8 à 14 et le radical alkyle représente un mélange d'au moins deux radicaux isomères.